# EUROPEAN PATENT APPLICATION

(11) **EP 2 554 533 A1**
(43) Date of publication of application: **06.02.2013**
(21) Application number: 11765527.4
(22) Date of filing: 28.03.2011
(51) Int. Cl.: C07C 49/92, C07D 207/333, C07D 213/22, C07D 333/22, C07D 471/04, H01L 51/50, H05B 33/12, C07F 5/00, C09K 11/06

(54) **RARE-EARTH METAL COMPLEX**

(30) Priority: 01.04.2010 JP 2010085483
(71) Applicant: Hitachi Chemical Company, Ltd., Tokyo 163-0449 (JP)
(72) Inventor: YAMASHITA, Takeshi, Tsukuba-shi Ibaraki 300-4247 (JP)
(74) Representative: HOFFMANN EITLE
(86) International application number: PCT/JP2011/057723
(87) International publication number: WO 2011/125627

(57) **Abstract**

A rare earth metal complex including a rare earth element and a β-diketone compound represented by the following formula (1) as a ligand, and in formula (1), R¹ represents a monovalent aromatic hydrocarbon group that may have a substituent or an aromatic heterocyclic group that may have a substituent.

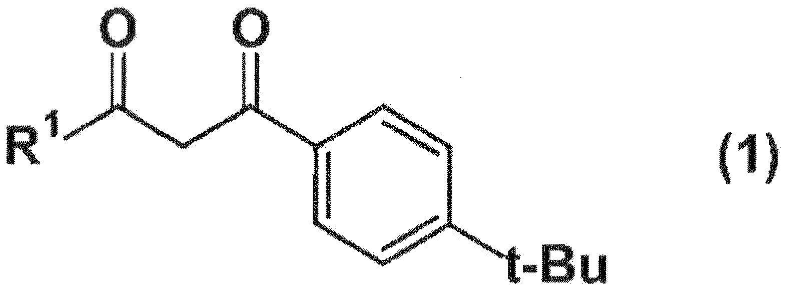

## Description

### Technical Field

The present invention relates to a rare earth metal complex which is excitable by excitation light having a longer wavelength than those of conventional ones, and is excellent in luminescence intensity.

### Background Art

In the past, various rare earth metal-based light emitting materials have been known, and the light emitting device in which light from an electric discharge lamp or a semiconductor light emitting device is color converted by luminescent material has been used for light devices or display devices.

In recent years, a luminescent material using a rare earth metal complex is particularly expected to be used in various field in that the solubility in solvent and dispersibility in resin are excellent, unlike in the case of inorganic luminescent material. For example, in the disclosures of Japanese Patent Application Laid-Open (JP-A) Nos. 2002-20358, 7-10819, 2008-19374, 2009-62335, 2009-292748, 2008-303196, 2007-71714 and the like, various proposals have been made for versatile purposes such as fluorescent probe, bioimaging, ink for printing, sensor, wavelength conversion resin sheet, and illumination.

As a luminescence mechanism of rare earth metal complex, a mechanism in which light is absorbed by a ligand, and the excitation energy transfers to the rare earth metal ion which is the luminescence center, whereby the ion is excited to emit light, is known. However, when the structure of the ligand is changed in order to shift an excitation wavelength toward a longer wavelength side, energy transfer efficiency between the ligand and the metal decreases so that practically sufficient luminescence intensity could not be obtained.

JP-A No. 2005-252250 suggests a rare earth metal complex excitable by exciting light having a longer wavelength than those of conventional ones, which is available by way of sufficient reduction of the deactivation caused by impurities, crystal defects or energy trap in the course of energy transfer from the ligand.

In addition, JP-A No. 2009-46577 suggests a rare earth metal complex excitable by exciting light having a longer wavelength than those of conventional ones, in which a rare earth metal complex coordinated with a phosphine oxide is reacted with a compound having a siloxane bond, thereby activating f-f transition of the rare earth metal.

### SUMMARY OF INVENTION

### Technical Problem

Under circumstances described above, for example, the rare earth metal complex described in JP-A No. 2005-252250 remains a matter of further improvement for luminescence intensity. In addition, the rare earth metal complex described in JP-A No. 2009-46577 is far from versatile in that hydrosilicone is required as an essential component.
The present invention was made to cope with the problem described above, and an object of the present invention is to provide a rare earth metal complex which is excitable by excitation light having a longer wavelength than those of conventional ones, and is excellent in luminescence intensity.

### Solution to Problem

The β-diketone ligand used for the rare earth metal complex has, particularly in the case in which the central metal is Eu³⁺, a maximum absorption wavelength of 350 nm or less, in most cases. Considering utility circumstances, it is desirable to shift an excitation wavelength of the rare earth metal complex toward a longer wavelength side.

Here, luminescence of the rare earth metal complex occurs by way of energy transfer from the ligand. In order to generate luminescence, in the relative relationship of energy level between the ligand and the central metal, it is necessary that the excitation level of the ligand is higher than that of the central metal. Therefore, to shift an excitation wavelength toward a longer wavelength side means narrowing the room for alternatives of possible energy transfer, which was, in principle, difficult.

However, the present inventor of the present invention conducted a thorough investigation, and as a result, the inventor found that when a β-diketone compound having particular structure is used in the rare earth metal as a ligand, a rare earth metal complex which is excitable by excitation light having a longer wavelength than those of conventional ones, and is excellent in luminescence intensity can be obtained.

Specifically, the present invention relates to the following.

<1> A rare earth metal complex comprising a rare earth element and a β-diketone compound represented by the following formula (1) as a ligand:

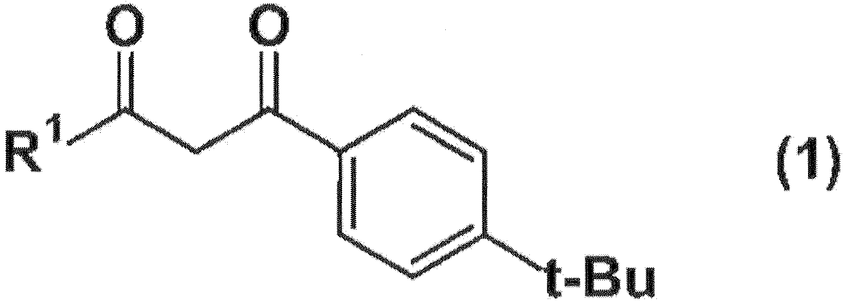

wherein in formula (1), R¹ represents a monovalent aromatic hydrocarbon group that may have a substituent or an aromatic heterocyclic group that may have a substituent.

<2> The rare earth metal complex according to item <1>, wherein the rare earth metal complex has a maximum absorption wavelength of 350 nm or longer and has a luminous efficacy of 50% or more at an excitation wavelength of 400 nm.

<3> The rare earth metal complex according to item <1> or item <2>, wherein the rare earth metal complex is represented by the following formula (2):

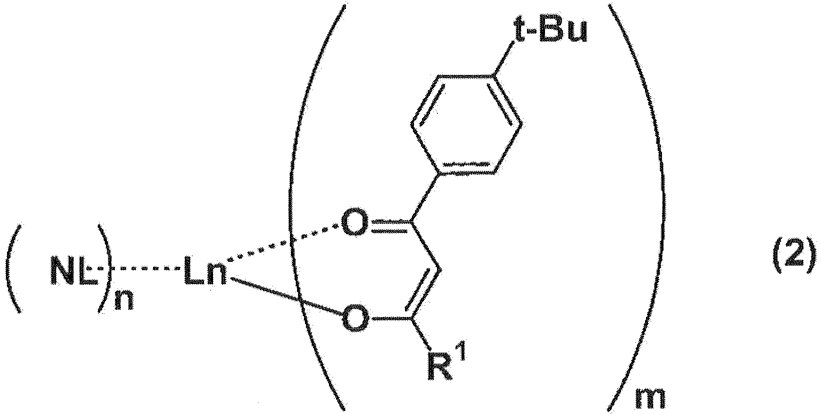

wherein in formula (2), Ln represents the rare earth element, NL represents a neutral ligand, R¹ represents the monovalent aromatic hydrocarbon group that may have a substituent or the aromatic heterocyclic group that may have a substituent, n represents an integer of from 1 to 5 and m is equivalent to a valence of Ln.

<4> The rare earth metal complex according to any one of items <1> to <3>, wherein the rare earth element is europium (Eu), terbium (Tb), erbium (Er), ytterbium (Yb), neodymium (Nd) or samarium (Sm).

### Advantageous Effects of Invention

According to the present invention, a rare earth metal complex which is excitable by excitation light having a longer wavelength than those of conventional ones, and is excellent in luminescence intensity, can be provided.

### BRIEF DESCRIPTION OF DRAWINGS

Fig. 1 shows the absorption spectra exhibiting the maximum values of the rare earth metal complexes obtained in Example 1, Comparative Example 1 and Comparative Example 2, respectively.
Fig. 2 shows the excitation spectra of the rare earth metal complexes obtained in Example 1, Example 2 and Comparative Example 3, respectively.
Fig. 3 is an enlarged view showing the emission spectra in the wavelength region of from 580 nm to 650 nm, with an excitation light of 400 nm, of the rare earth metal complexes obtained in Example 1, Comparative Example 1 and Comparative Example 3, respectively.

### DESCRIPTION OF EMBODIMENTS

A rare earth metal complex of the present invention is a complex which includes a rare earth element and a β-diketone compound represented by the following formula (1) as a ligand: 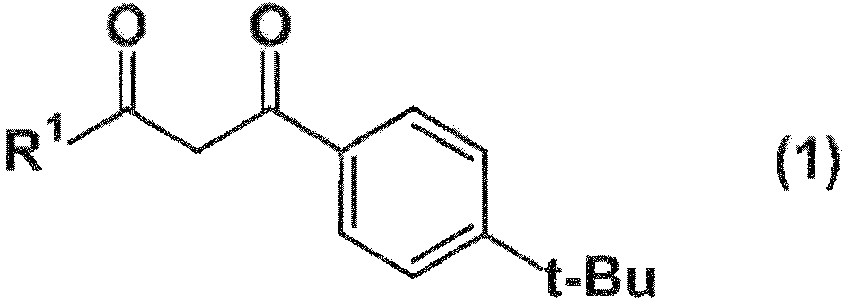 wherein in formula (1), R¹ represents a monovalent aromatic hydrocarbon group that may have a substituent or an aromatic heterocyclic group that may have a substituent.

Examples of the aromatic hydrocarbon group include a benzene ring group, a naphthalene ring group, an anthracene ring group, a phenanthrene ring group, a pyrene ring group, a perylene ring group, a tetracene ring group, a chrysene ring group, a pentacene ring group, a triphenylene ring group, an indene ring group and an azulene ring group.

Examples of the aromatic heterocyclic group include a pyrrole ring group, a thiophene ring group, a furan ring group, an imidazole ring group, a pyrazole ring group, a pyridine ring group, a pyridazine ring group, a pyrimidine ring group, a pyrazine ring group, a triazole ring group, a triazine ring group, a thiazole ring group, an isothiazole ring group, an oxazole ring group, an isoxazole ring group, an indole ring group, an isoindole ring group, a benzofuran ring group, an isobenzofuran ring group, a benzoxazole ring group, a benzisoxazole ring group, a benzothiazole ring group, a benzothiophene ring group and a carbazole ring group.

The monovalent aromatic hydrocarbon group and aromatic heterocyclic group represented by R¹ may be unsubstituted or may have a substituent. Examples of the substituent include an alkyl group, an alkoxy group, a halogen group, a perfluoroalkyl group, a nitro group, an amino group, a sulfonyl group, a cyano group, a silyl group, a phosphonic group, a diazo group, a mercapto group, an aryl group, an aralkyl group, an aryloxy goup, an aryloxycarbonyl group, an allyl group, an acyl group and an acyloxy group. Preferred examples thereof include an alkyl group, an alkoxy group, a halogen group and a perfluoroalkyl group; and an alkyl group having 1 to 4 carbon atoms, an alkoxy group having 1 to 4 carbon atoms and a perfluoroalkyl group having 1 to 3 carbon atoms are more preferred. Specifically, preferred examples thereof include a methyl group, an ethyl group, a propyl group, an isopropyl group, a butyl group, a methoxy group, an ethoxy group, a propoxy group, an isopropoxy group, a butoxy group, a trifluoromethyl group, a pentafluoroethyl group and a heptafluoropropyl group. Among these, a methyl group, an ethyl group, a propyl group, an isopropyl group, a methoxy group, an ethoxy group, a propoxy group, an isopropoxy group, a trifluoromethyl group, a pentafluoroethyl group or a heptafluoropropyl group is more preferred; and a methyl group, an ethyl group, a propyl group, an isopropyl group, a methoxy group, an ethoxy group, a propoxy group or an isopropoxy group is still more preferred.

When the aromatic hydrocarbon group and aromatic heterocyclic group represented by R¹ have a substituent, the number of the substituent is not limited. In the case of having 1 to 5 substituents is preferred. In the case of having 1 to 3 substituents is more preferred, and in the case of having 1 to 2 substituents is still more preferred.

Further, when the aromatic hydrocarbon group and aromatic heterocyclic group represented by R¹ have a substituent, the substituted position of the substituent is not limited. When the aromatic hydrocarbon group represented by R¹ is a phenyl group, any of ortho-position, meta-position or para-position may be substituted, and it is more preferred to have a substituent at para-position.

Among above described groups, preferred examples of R¹ include a phenyl group having an alkoxy group or a pyrrol group having an alkyl group, more preferably a phenyl group having an alkoxy group at para-position or a N-alkyl-3-pyrrol group, and still more preferably a phenyl group having an alkoxy group having 1 to 3 carbon atoms at para-position or a N-alkyl having 1 to 3 carbon atoms -3-pyrrol group.

The following are specific examples of β-diketone compound represented by formula (1). However, the present invention is not limited to these specific examples.

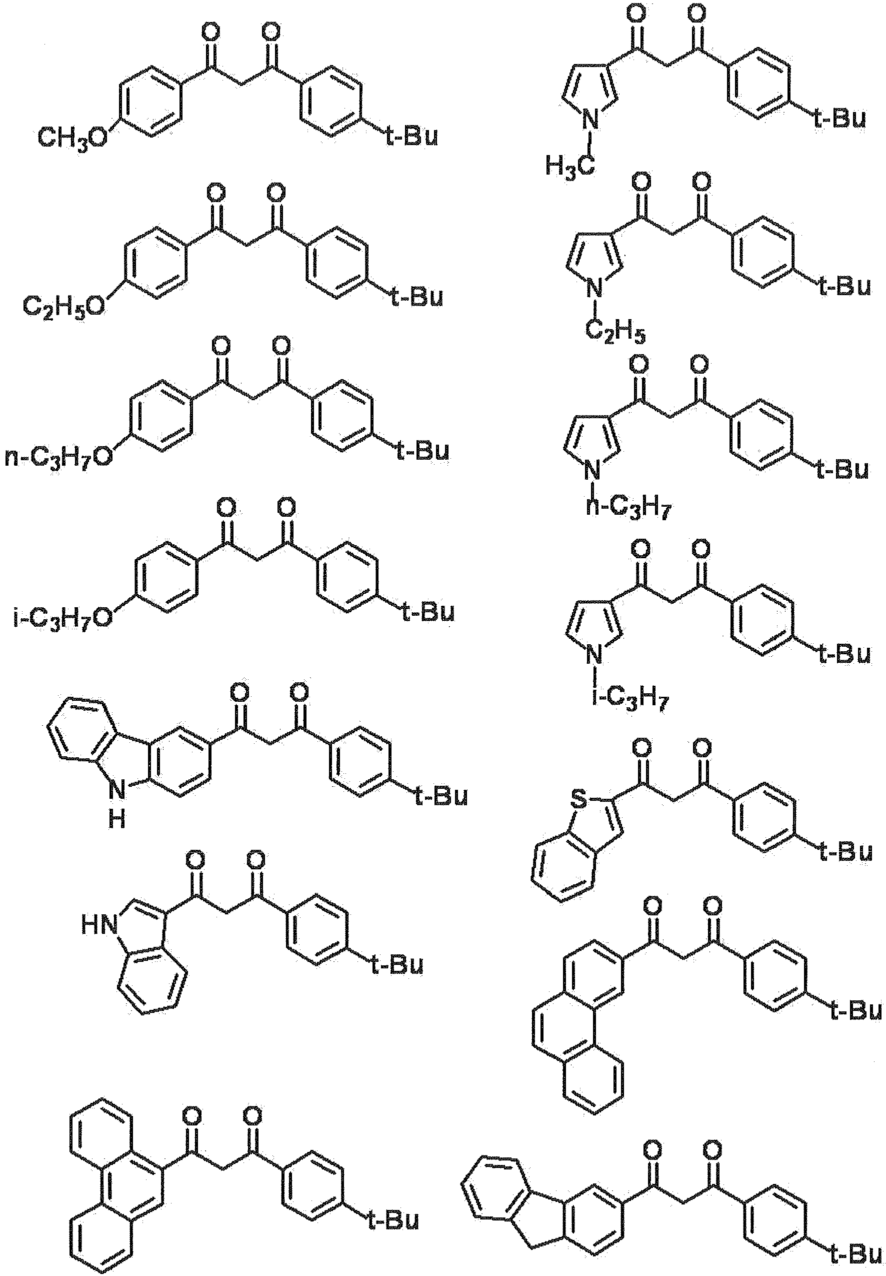

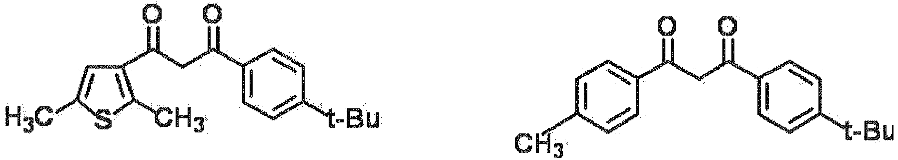

The β-diketone compound represented by formula (1) may be obtained by, for example as shown in the following reaction formula, condensing aromatic ketons with 4-t-butyl benzoate esters in the presence of a base.

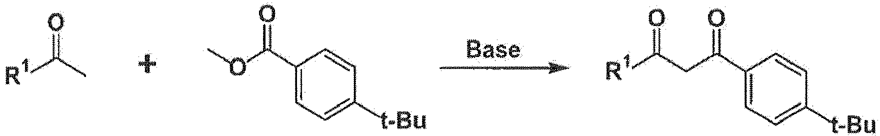

The rare earth element in the rare earth metal complex of the present invention is preferably europium (Eu), terbium (Tb), erbium (Er), ytterbium (Yb), neodymium (Nd) or samarium (Sm). Eu, Sm or Tb is more preferred and Eu is particularly preferred.

The rare earth metal complex of the present invention in which a β-diketone is included as a ligand is not limited as long as the total ligancy with respect to the rare earth element is in a range of from 6 to 9. For example, a complex in which three molecules of β-diketonate which serves as an anion having a minus monovalence are coordinated with respect to a rare earth metal ion having a plus trivalence; and a complex in which a neutral ligand having Lewis basicity is coordinated as an auxiliary ligand with the complex described above; or a complex in which four molecules of β-diketonate are coordinated, and having a cationic molecule so that the valency as a whole is neutralized, may be included.
Particularly, in view of dispersibility to a medium and fluorescent property as a fluorescent material, a complex which includes three molecules of β-diketonate with respect to a rare earth metal and a neutral ligand which is a Lewis base, is preferred.

The rare earth metal complex of the present invention is preferably a complex represented by the following formula (2).

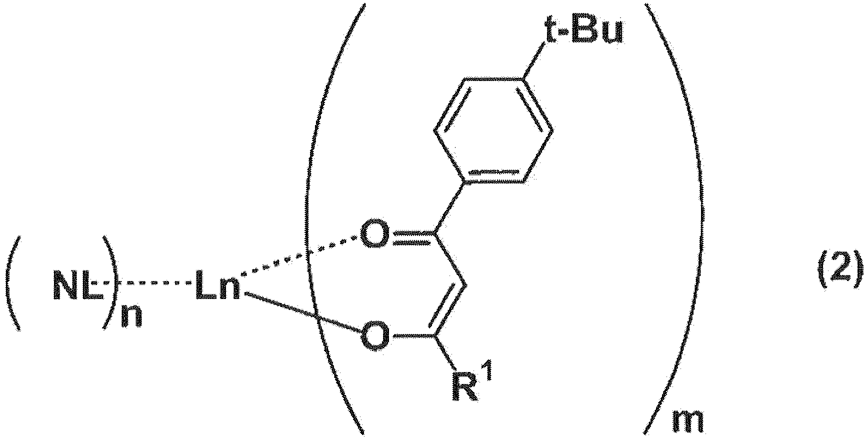

In formula (2), Ln represents a rare earth element; NL represents a neutral ligand; R¹ represents the monovalent aromatic hydrocarbon group that may have a substituent or the aromatic heterocyclic group that may have a substituent; n represents an integer of from 1 to 5 and m is equivalent to a valence of Ln.

In formula (2), examples of the rare earth element represented by Ln include those previously mentioned, and preferred rare earth elements are also the same as previously mentioned so that the explanation here is omitted.

The neutral ligand represented by NL is not particularly limited, which is a compound capable of being coordinated with Ln and has at least one selected from a nitrogen atom, an oxygen atom and a sulfur atom. Examples thereof include an amine, an amine oxide, a phosphine oxide, a ketone, a sulfoxide and an ether, which may be selected alone or may be selected in combination thereof.
In addition, when Ln is Eu³⁺, the neutral ligand is selected so that the total ligancy of Eu³⁺ is 7, 8 or 9.

Examples of amine represented by neutral ligand NL include, for example, pyridine, pyrazine, quinoline, isoquinoline, 2,2'-bipyridine and 1,10-phenanthroline, which may have a substituent.
Examples of amine oxide represented by neutral ligand NL include, for example, N-oxide of the above described amine such as pyridine-N-oxide, isoquinoline-N-oxide, 2,2'-bipyridine-N,N'-dioxide and 1,10-phenanthroline-N,N'-dioxide, which may have a substituent.

Examples of phosphine oxide represented by neutral ligand NL include, for example, triphenyl phosphine oxide which may have a substituent, alkylalkyl phosphine oxide, such as triethyl phosphine oxide and trioctyl phosphine oxide, 1,2-ethylenebis (diphenylenephosphine oxide), (diphenylphosphine imid) triphenylphosphorane, and triphenyl phosphate.

Examples of ketone represented by neutral ligand NL include, for example, dipyridylketone and benzophenone, which may optionally have a substituent.

Examples of sulfoxide represented by neutral ligand NL include, for example, diphenylsulfoxide, dibenzylsulfoxide and dioctylsulfoxide, which may have a substituent.

Examples of ether represented by neutral ligand NL include, for example, ethylene glycol dimethy ether and ethylene glycol dimethy ether, which may have a substituent.

In formula (2), n represents an integer of from 1 to 5, preferably an integer of from 1 to 3, and more preferably an integer of from 1 to 2.

In formula (2), m is equivalent to a valence of Ln. For example, when Ln is Eu³⁺, m is 3.

In formula (2), when the rare earth element Ln is Eu, the neutral ligand NL is preferably an amine, a phosphine oxide or a sulfoxide, more preferably an amine or a phosphine oxide, and still more preferably an amine. In addition, among amines, the neutral ligand NL represented by the following formula (3) is preferable.

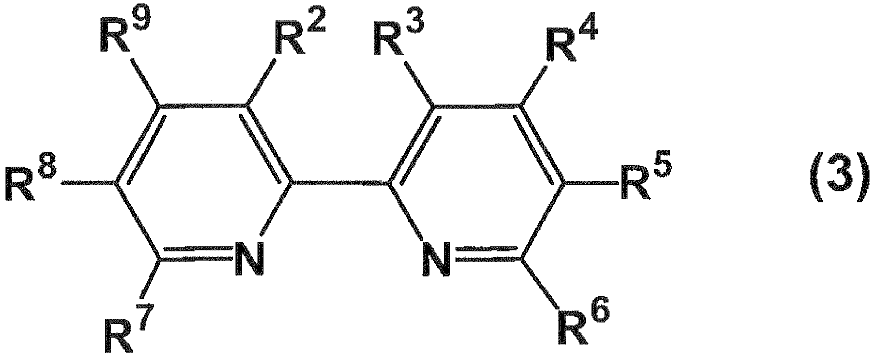

In formula (3), R² to R⁹ each independently represent a hydrogen atom, an alkyl group or an aryl group. In addition, R² and R³, R³ and R⁴, R⁴ and R⁵, R⁵ and R⁶, R⁷ and R⁸, R⁸ and R⁹ and R⁹ and R² may be bonded to each other to form a ring.

Formula (3) is preferably a bipyridine compound in which R² and R³ in formula (3) each independently represent a hydrogen atom or a phenanthroline compound in which R² and R³ in formula (3) are bonded to each other to form a benzene ring.

R² to R⁹ in formula (3) each independently represent preferably a hydrogen atom, an alkyl group having 1 to 9 carbon atoms or a phenyl group, more preferably a hydrogen atom, a methyl group, an ethyl group or a phenyl group, and still more preferably a hydrogen atom, a methyl group or a phenyl group.

In formula (3), in the case in which any of R⁴ to R⁹ is an alkyl group or an aryl group, it is preferable that at least R⁵ or R⁸ (that is, fifth position) is an alkyl group or an aryl group.

Specific examples of preferred neutral ligand NL represented by formula (3) include 2,2'-bipyridine, 1,10-phenanthroline, bathophenanthroline, neocuproin, bathocuproin, 5,5'-dimethyl-2,2'-bipyridine, 4,4'-dimethyl-2,2'-bipyridine, 6,6'-dimethyl-2,2'-bipyridine, 5-phenyl-2,2'-bipyridine, 2,2'-biquinoline, 2,2'-bi-4-lepidine, 2,9-dibutyl-1,10-phenanthroline, 3,4,7,8-tetramethyl-1,10-phenanthroline and 2,9-dibutyl-1,10-phenanthroline. 2,2'-Bipyridine, 1,10-phenanthroline, bathophenanthroline, 5,5'-dimethyl-2,2'-bipyridine and 5-phenyl-2,2'-bipyridine are more preferred.

In addition, in formula (2), when the rare earth element Ln is Eu, n is preferably an integer of from 1 to 2 and more preferably n is 1.

The rare earth metal complex of the present invention may be easily obtained by reacting a rare earth metal compound with a β-diketone in the presence of a base.

The rare earth metal compound used for producing the rare earth metal complex is not particularly limited as long as it is easily obtained in general. Examples thereof include an inorganic compound such as an oxide, a hydroxide, a sulfide, a fluoride, a chloride, a bromide, an iodide, a sulfate, a sulfite, a disulfate, a hydrogen sulfate, a thiosulfate, a nitrate, a nitrite, a phosphate, a phosphite, a hydrogen phosphate, a dihydrogen phosphate, a diphosphate, a polyphosphate, a (hexa)fluorophosphate, a carbonate, a hydrogen carbonate, a thiocarbonate, a cyanite, a thiocyanate, a borate, a tetrafluoroborate, a cyanate, a thiocyanate, an isothiocyanate, an azide, a nitride, a boride, a silicate, a (hexa)fluorosilicate of a rare earth metal, and a rare earth metal salt of isopoly acid, heteropoly acid or other condensed polyacid; and an organic compound such as an alcoholate, a thiolate, an amide, an imide, a carboxylate, a sulfonate, a phosphonate, a phosphinate, an amino acid salt, a carbamate and a xanthogenate of a rare earth metal.

The rare earth metal complex of the present invention preferably has a maximum absorption wavelength of 350 nm or longer, more preferably in a range of from 350 nm to 400 nm, and still more preferably in a range of from 355 nm to 375 nm.
The maximum absorption wavelength of the rare earth metal complex of the present invention corresponds to a wavelength attributed to a β-diketone compound. When the rare earth element is coordinated with a β-diketone compound, the absorption wavelength is observed as that of anion of the β-diketone, that is, as that of the β-diketonate. In order to shift the absorption wavelength of β-diketones toward a longer wavelength side, it is preferable to elongate the conjugate system.

The maximum absorption wavelength of the rare earth metal complex of the present invention may be measured by using a commercially available spectrophotometer (for example, U-3310 manufactured by Hitachi High-Tech Fielding Corporation) and by using a square quartz cell of 1 cm optical length in the solution which is adjusted so that the absorbance is 1 or less. As a measuring solvent, those high in solubility of the sample and low in absorption at ultraviolet area are preferable. Examples of such a solvent include tetrahydrofuran and dimethylformamide. In addition, measured concentration varies depending on the molar absorption coefficient of each of the samples, and it is adjusted so that the absorbance is in the range of from 0.1 to 1.0. In the present invention, the value measured by using dimethylformamide as a solvent at a concentration of 2x 10⁻⁵ M is adopted.

In addition, the rare earth metal complex of the present invention has a maximum excitation wavelength preferably from 395 nm to 450 nm, more preferably from 400 nm to 440 nm and still more preferably from 405 nm to 435 nm.

The maximum excitation wavelength of the rare earth metal complex of the present invention may be measured by using a commercially available spectrofluorophotometer (for example, F-4500 manufactured by Hitachi High-Technologies Corporation), fixing the spectrometer of the fluorescence side (specifically, when the luminescence center is Eu³⁺, it is appropriately adjusted between 605 nm to 620 nm in which the maximum luminescence intensity is exhibited), and scanning the spectrometer of the excitation side. As a sample form, it is selected from powder, solution, resin dispersion state and the like. In relative comparison, any of these forms may be available. In addition, in the case of powder state, scattering may occur and in the case of solution/resin dispersing state, an effect of medium and concentration dependency may occur, which requires an attention. The maximum excitation wavelength in the present invention adopts the value measured by using dimethylformamide as a solvent at a concentration of 1×10⁻⁴ M.

Further, in the rare earth metal complex of the present invention, the luminescent efficiency at the excitation wavelength of 400 nm is preferably 50% or more, more preferably 55% or more, and still more preferably 60% or more.

The method for obtaining luminescent efficiency and luminescence intensity of the rare earth metal complex of the present invention will be explained.

The rare earth metal complex (fluorescent material) to be measured is placed in the integrating sphere equipped with a spectrophotometer and an excitation light source, to which the light of 400 nm is exposed from the excitation light source and measurement is carried out. Examples of the measurement device include QEMS2000 manufactured by Systems Engineering Inc. The reason for using an integrating sphere or the like is such that the entire photons including those reflected by the sample and those emitted from the sample due to photoluminescence can be counted.

In the measurement spectrum, other than photons emitted from the sample by the light from the excitation light source due to photoluminescence, contribution of photons of the excitation light reflected by the sample is practically overlapping. Accordingly, luminescent efficiency shall be the value of the number of photons emitted by the sample due to photoluminescence divided by the total number of photons of the excitation light absorbed by the sample.

In addition, when the excitation light intensity is set constant, luminescent efficiency shall be the sum of the number of photons emitted by the sample due to photoluminescence. Further, when the central metal is europium ion (Eu³⁺), integral interval may be from 550 nm to 750 nm which includes a range of from 600 nm to 630 nm, the range is derived from ⁵D₀ to ⁷F₂ transition which is the most intense emission wavelength region

The rare earth metal complex of the present invention can be applied to, for example, a wavelength conversion resin composition which is applied at the side of light receiving surface of the photovoltaic cell, a wavelength conversion photovoltaic cell sealing material (a wavelength conversion photovoltaic cell sealing sheet), and a photovoltaic cell module using the same. When the rare earth metal complex of the present invention is applied to these usages, the light having wavelength region which contributes to slight generation of electricity may be wavelength converted to the light having wavelength region which contributes to significant generation of electricity so that the generating efficiency is improved.

The disclosure of Japanese Patent Application No. 2010-085483 is hereby incorporated by reference in its entirety in the present application.

All publications, patent applications, and technical standards mentioned in this specification are herein incorporated by reference to the same extent as if each individual publication, patent application, or technical standard was specifically and individually indicated to be incorporated by reference.

### EXAMPLES

Hereinafter, the present invention will be more specifically described by way of Examples, but the present invention is not intended to be limited to these Examples.

### [Example 1]

### <Synthesis of BMPP(1-(p-t-butylphenyl)-3-(N-methyl-3-pyrrol)-1,3-propanedione)>

1.92 g (0.08 mol) of Sodium hydride was weighed, and 45 ml of dehydrated tetrahydrofuran was added thereto under a nitrogen atmosphere. While the mixture was vigorously stirred, a solution prepared by dissolving 4.93 g (0.04 mol) of 3-acetyl-1-methylpyrrol and 4.93 g (0.04 mol) of methyl-4-t-butylbenzoate in 50 ml of dehydrated tetrahydrofuran was added dropwise to the mixture over one hour. Thereafter, the resultant mixture was refluxed for 8 hours. Subsequently, the mixture was cooled to room temperature, then 20 g of pure water was added thereto, and 16.5 ml of 3N hydrochloric acid was further added thereto. The organic layer was separated, and concentrated under reduced pressure. The concentrate was recrystallized, and thus, 8.73 g (yield 77%) of BMPP as white powder was obtained.

### <Synthesis of Eu(BMPP)₃Phen>

643.7 mg (2.24 mmol) of thus synthesized BMPP and 151.4 mg (0.84 mmol) of 1,10-phenanthroline (Phen) were dispersed in 25.0 g of methanol. To the dispersion liquid, a solution prepared by dissolving 109.2 mg (2.73 mmol) of sodium hydrate in 10.0 g of methanol was added, and stirred for one hour.
Subsequently, a solution prepared by dissolving 256.5 mg (0.7 mmol) of europium (III) chloride hexahydrate in 5.0 g of methanol was added dropwise to the mixture. The resultant mixture was stirred for one hour at room temperature. Subsequently, the mixture was heated at 60°C in the oil bath, and further stirred for two hours. Then the resultant mixture was cooled to room temperature, and produced precipitate was suction filtrated, washed with methanol, and dried to obtain Eu (BMPP)₃Phen.

### [Example 2]

### <Synthesis of Eu(BMDBM)₃Phen>

695.3 mg (2.24 mmol) of 1-(p-t-butylphenyl)-3-(p-methoxyphenyl)-1,3-propanedione (BMDBM) and 151.4 mg (0.84 mmol) of 1,10-phenanthroline (Phen) were dissolved in 25.0 g of methanol. To the solution, a solution prepared by dissolving 109.2 mg (2.73 mmol) of sodium hydrate in 10.0 g of methanol was added dropwise, and stirred for one hour.
Subsequently, a solution prepared by dissolving 256.5 mg (0.7 mmol) of europium (III) chloride hexahydrate in 5.0 g of methanol was added dropwise to the mixed solution, and the resultant solution was continued to be stirred for two hours. The produced precipitate was suction filtrated, washed with methanol, and dried to obtain Eu(BMDBM)₃Phen.

### [Example 3]

### <Synthesis of BDMTP>

### (1-(p-t-butylphenyl)-3-(2,5-dimethyl-3-thienyl)-1,3-propanedione)>

0.96 g (0.04 mol) of Sodium hydride was weighed, and 22.5 ml of dehydrated tetrahydrofuran was added thereto under a nitrogen atmosphere. While the mixture was vigorously stirred, a solution prepared by dissolving 3.08 g (0.02 mol) of 3-acetyl-2,5-dimethylthiophene and 4.61 g (0.024 mol) of methyl-4-t-butylbenzoate in 25 ml of dehydrated tetrahydrofuran was added dropwise to the mixture over one hour. Thereafter, the resultant mixture was refluxed for 8 hours. Subsequently, the resultant mixture was cooled to room temperature, then 10 g of pure water was added thereto, and 8.0 ml of 3N hydrochloric acid was further added thereto. The organic layer was separated, and concentrated under reduced pressure. The concentrate was recrystallized, and thus, 4.58 g (yield 73%) of BDMTP as white powder was obtained.

### <Synthesis of Eu(BDMTP)₃Phen>

704.3 mg (2.24 mmol) of thus synthesized BDMTP and 151.4 mg (0.84 mmol) of 1,10-phenanthroline (Phen) were dispersed in 25.0 g of methanol. To the dispersion liquid, a solution prepared by dissolving 112.0 mg (2.80 mmol) of sodium hydrate in 10.0 g of methanol was added, and stirred for one hour.
Subsequently, a solution prepared by dissolving 256.5 mg (0.7 mmol) of europium (III) chloride hexahydrate in 5.0 g of methanol was added dropwise to the mixture. The resultant mixture was stirred for two hours at room temperature. Subsequently, the produced precipitate was suction filtrated, washed with methanol, and dried to obtain Eu(BDMTP)₃Phen.

### [Example 4]

### <Synthesis of BMeP (1-(p-t-butylphenyl)-3-(4-methylphenyl)-1,3-propanedione)>

0.96 g (0.04 mol) of Sodium hydride was weighed, and 22.5 ml of dehydrated tetrahydrofuran was added thereto under a nitrogen atmosphere. While the mixture was vigorously stirred, a solution prepared by dissolving 2.68 g (0.02 mol) of 4-methylacetophenone and 4.61 g (0.024 mol) of methyl-4-t-butylbenzoate in 25 ml of dehydrated tetrahydrofuran was added dropwise to the mixture over one hour. Thereafter, the resultant mixture was refluxed for 6 hours. Subsequently, the resultant mixture was cooled to room temperature, then 10 g of pure water was added thereto, and 5.0 ml of 3N hydrochloric acid was further added thereto. The organic layer was separated, and concentrated under reduced pressure. The concentrate was recrystallized, and thus, 1.87 g (yield 32%) of BMeP as white powder was obtained.

### <Synthesis of Eu(BMeP)₃Phen>

470.4 mg (1.6 mmol) of thus synthesized BMeP and 108.1 mg (0.6 mmol) of 1,10-phenanthroline (Phen) were dispersed in 17.9 g of methanol. To the dispersion liquid, a solution prepared by dissolving 80.8 mg (2.0 mmol) of sodium hydrate in 7.1 g of methanol was added, and stirred for one hour.
Subsequently, a solution prepared by dissolving 183.2 mg (0.5 mmol) of europium (III) chloride hexahydrate in 3.6 g of methanol was added dropwise to the mixture. The resultant mixture was stirred for two hour at room temperature. Subsequently, the produced precipitate was suction filtrated, washed with methanol, and dried to obtain Eu(BMeP)₃Phen.

### [Example 5]

### <Synthesis of Eu(BMDBM)₃Bpy>

695.3 mg (2.24 mmol) of 1-(p-t-butylphenyl)-3-(p-methoxyphenyl)-1,3-propanedione (BMDBM) and 131.2 mg (0.84 mmol) of 2,2'-bipyridine (Bpy) were dissolved in 25.0 g of methanol. To the solution, a solution prepared by dissolving 109.2 mg (2.73 mmol) of sodium hydrate in 10.0 g of methanol was added dropwise, and then stirred for one hour.
Subsequently, a solution prepared by dissolving 256.5 mg (0.7 mmol) of europium (III) chloride hexahydrate in 5.0 g of methanol was added dropwise to the mixture, and the resultant mixture was continued to be stirred for two hours. The produced precipitate was suction filtrated, washed with methanol, and dried to obtain Eu(BMDBM)₃Bpy.

### [Example 6]

### <Synthesis of Eu(BMDBM)₃5dm-Bpy>

695.3 mg (2.24 mmol) of BMDBM and 154.8 mg (0.84 mmol) of 5,5'-dimethyl-2,2'-bipyridine (5dm-Bpy) were dispersed in 25.0 g of methanol. To the liquid, a solution prepared by dissolving 109.2 mg (2.73 mmol) of sodium hydrate in 10.0 g of methanol was added dropwise, and then stirred for one hour.
Subsequently, a solution prepared by dissolving 256.5 mg (0.7 mmol) of europium (III) chloride hexahydrate in 5.0 g of methanol was added dropwise to the mixture, and the resultant mixture was continued to be stirred for two hours. The produced precipitate was suction filtrated, washed with methanol, and dried to obtain Eu(BMDBM)₃5dm-Bpy.

### [Example 7]

### <Synthesis of Eu(BMDBM)₃5p-Bpy>

695.3 mg (2.24 mmol) of BMDBM and 195.1 mg (0.84 mmol) of 5-phenyl-2,2'-bipyridine (5p-Bpy) were dispersed in 25.0 g of methanol. To the liquid, a solution prepared by dissolving 109.2 mg (2.73 mmol) of sodium hydrate in 10.0 g of methanol was added dropwise, and then stirred for one hour.
Subsequently, a solution prepared by dissolving 256.5 mg (0.7 mmol) of europium (III) chloride hexahydrate in 5.0 g of methanol was added dropwise to the mixture, and the resultant mixture was continued to be stirred for two hours. The produced precipitate was suction filtrated, washed with methanol, and dried to obtain Eu(BMDBM)₃5p-Bpy.

### [Example 8]

### <Synthesis of Eu(BMDBM)₃4dm-Bpy>

695.3 mg (2.24 mmol) of BMDBM and 154.8 mg (0.84 mmol) of 4,4'-dimethyl-2,2'-bipyridine (4dm-Bpy) were dissolved in 25.0 g of methanol. To the liquid, a solution prepared by dissolving 109.2 mg (2.73 mmol) of sodium hydrate in 10.0 g of methanol was added dropwise, and then stirred for one hour.
Subsequently, a solution prepared by dissolving 256.5 mg (0.7 mmol) of europium (III) chloride hexahydrate in 5.0 g of methanol was added dropwise to the mixture, and continued to stirr for two hours. The produced precipitate was suction filtrated, washed with methanol, and dried to obtain Eu(BMDBM)₃4dm-Bpy.

### [Comparative Example 1]

### <Synthesis of Eu(TTA)₃Phen>

A solution prepared by dissolving 2.00 g (9.00 mmol) of thenoyltrifluoroacetone (TTA) in 75.0 g of ethanol was added to 11 g of aqueous sodium hydroxide (1M). Subsequently, a solution prepared by dissolving 0.62 g (3.44 mmol) of 1,10-phenanthroline in 75.0 g of ethanol was added to the mixture, and then the resultant mixture was stirred for one hour.
Subsequently, a solution prepared by dissolving 1.03 g (2.81 mmol) of europium (III) chloride hexahydrate in 20.0 g of ethanol was added dropwise to the mixture, and the resultant mixture was continued to be stirred for one hour. The produced precipitate was suction filtrated, washed with ethanol, and dried to obtain Eu(TTA)₃Phen.

### [Comparative Example 2]

### <Synthesis of Eu (BFA)₃Phen>

A solution prepared by dissolving 1.94 g (9.00 mmol) of benzoyltrifluoroacetone (BFA) in 60.0 g of ethanol was added to 11 g of aqueous sodium hydroxide (1M). Subsequently, a solution prepared by dissolving 0.62 g (3.44 mmol) of 1,10-phenanthroline in 60.0 g of ethanol was added to the mixture, and then the resultant mixture was stirred for one hour.
Subsequently, a solution prepared by dissolving 1.03 g (2.81 mmol) of europium (III) chloride hexahydrate in 20.0 g of ethanol was added dropwise to the mixture, and the resultant mixture was continued to be stirred for one hour. The produced precipitate was suction filtrated, washed with ethanol, and dried to obtain Eu (BFA)₃Phen.

### [Comparative Example 3]

### <Synthesis of Eu (DBM)₃Phen>

A solution prepared by dissolving 2.00 g (9.00 mmol) of dibenzoylmethane (DBM) in 60.0 g of ethanol was added to 11 g of aqueous sodium hydroxide (1M). Subsequently, a solution prepared by dissolving 0.62 g (3.44 mmol) of 1,10-phenanthroline in 60.0 g of ethanol was added to the mixture, and then the resultant mixture was stirred for one hour.
Subsequently, a solution prepared by dissolving 1.03 g (2.81 mmol) of europium (III) chloride hexahydrate in 20.0 g of ethanol was added dropwise to the mixture, and the resultant mixture was continued to be stirred for one hour. The produced precipitate was suction filtrated, washed with ethanol, and dried to obtain Eu (DBM)₃Phen.

### [Method of measurement]

In the following, the method of measuring each of the parameters such as excitation wavelength measured in each of the examples will be explained.

### 1. Measurement of maximum absorption wavelength

The measurement was carried out by using U-3310 manufactured by Hitachi High-Tech Fielding Corporation, as a spectrophotometer.

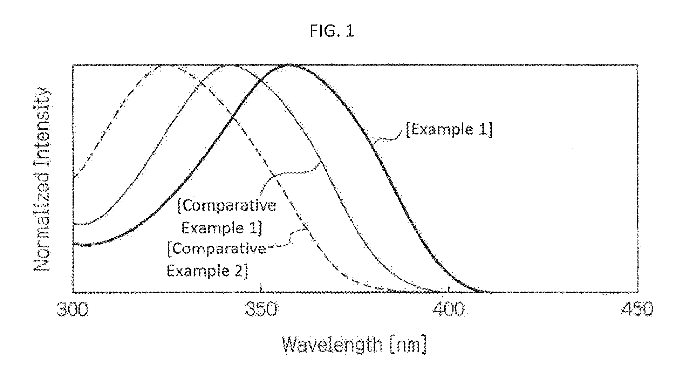

Fig. 1 shows the absorption spectra exhibiting the maximum values of the rare earth metal complexes obtained in Example 1, Comparative Example 1 and Comparative Example 2, respectively.

### 2. Measurement of maximum excitation wavelength

The measurement was carried out by using F-4500 manufactured by Hitachi High-Technologies Corporation, as a spectrofluorophotometer.

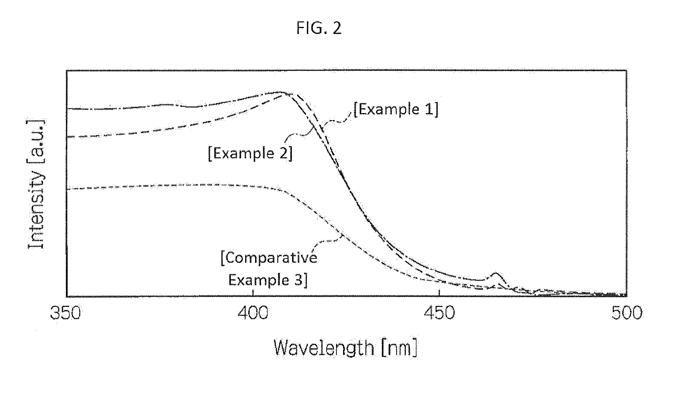

Fig. 2 shows the excitation spectra of the rare earth metal complexes obtained in Example 1, Example 2 and Comparative Example 3, respectively.

### 3. Measurement of luminescence intensity and luminescent efficiency

The measurement was carried out by using QEMS2000 manufactured by Systems Engineering Inc, as a measurement device for luminescent quantum efficiency. Excitation light of 400 mn was irradiated to the sample, and luminescent efficiency was measured. In addition, based on the emission spectrum, the sum of the number of photons in the integral interval of from 550 mn to 750 nm was regarded as luminescence intensity.

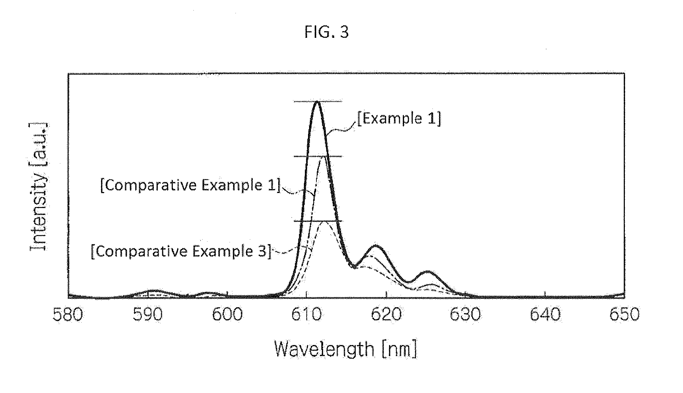

Fig. 3 shows an enlarged view of the emission spectra in the wavelength region of from 580 nm to 650 nm, with an excitation light of 400 nm, of the rare earth metal complexes obtained in Example 1, Comparative Example 1 and Comparative Example 3, respectively.

**[Table 1]**

| | Rare earth metal complex | Maximum absorption wavelength (nm) | Maximum excitation wavelength (nm) | Luminescence intensity | Luminescent efficiency (%) |
|---|---|---|---|---|---|
| Example 1 | Eu(BMPP)₃Phen | 358 | 414 | 131 | 60 |
| Example 2 | Eu(BMDBM)₃Phen | 358 | 416 | 122 | 56 |
| Example 3 | Eu(BDMTP)₃Phen | 355 | 414 | 134 | 62 |
| Example 4 | Eu(BMeP)₃Phen | 356 | 417 | 93 | 42 |
| Example 5 | Eu(BMDBM)₃Bpy | 358 | 416 | 113 | 51 |
| Example 6 | Eu(BMDBM)₃5dm-Bpy | 358 | 417 | 109 | 49 |
| Example 7 | Eu(BMDBM)₃5p-Bpy | 358 | 418 | 99 | 47 |
| Example 8 | Eu(BMDBM)₃4dm-Bpy | 358 | 418 | 63 | 29 |
| Comparative Example 1 | Eu(TTA)₃Phen | 342 | 391 | 100 | 67 |
| Comparative Example 2 | Eu(BFA)₃Phen | 325 | 375 | 74 | 62 |
| Comparative Example 3 | Eu(DBM)₃Phen | 353 | 415 | 61 | 29 |

As shown in Table 1, it can be seen that the rare earth metal complexes of Examples 1 to 8 which include a β-diketone compound represented by formula (1) as a ligand are excited by excitation light having a longer wavelength compare to the rare earth metal complexes of Comparative Examples 1 to 2 which do not include a β-diketone compound represented by formula (1) as a ligand. In addition, it can be seen that the luminescence intensity is superior to that of Comparative Example 3.

## Claims

1. A rare earth metal complex comprising a rare earth element and a β-diketone compound represented by the following formula (1) as a ligand: 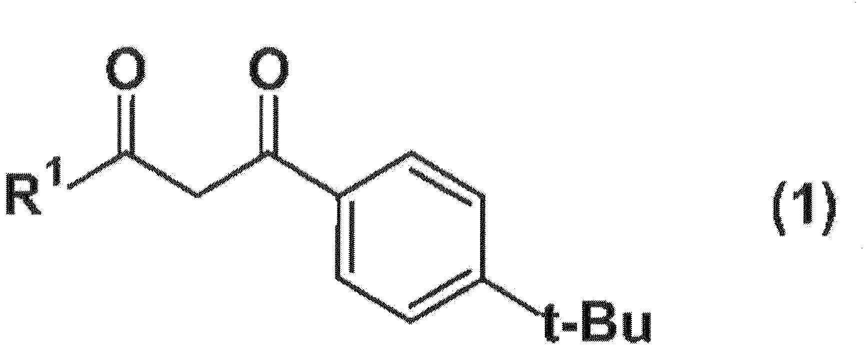 wherein in formula (1), R¹ represents a monovalent aromatic hydrocarbon group that may have a substituent or an aromatic heterocyclic group that may have a substituent.

2. The rare earth metal complex according to claim 1, wherein the rare earth metal complex has a maximum absorption wavelength of 350 nm or longer and has a luminous efficacy of 50% or more at an excitation wavelength of 400 nm.

3. The rare earth metal complex according to claim 1 or claim 2, wherein the rare earth metal complex is represented by the following formula (2): 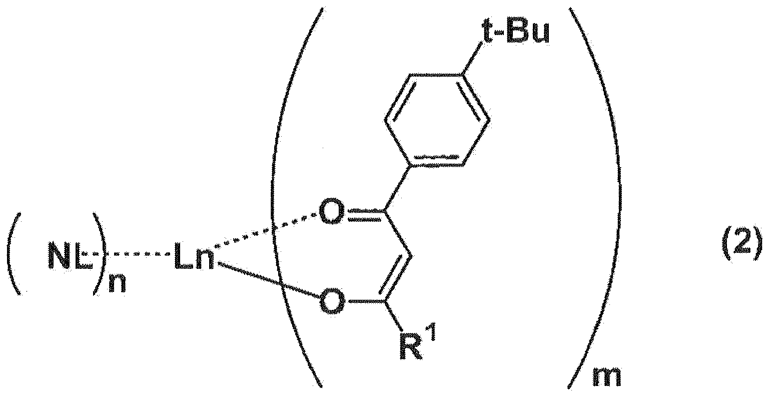 wherein in formula (2), Ln represents the rare earth element; NL represents a neutral ligand; R¹ represents the monovalent aromatic hydrocarbon group that may have a substituent or the aromatic heterocyclic group that may have a substituent; n represents an integer of from 1 to 5 and m is equivalent to a valence of Ln.

4. The rare earth metal complex according to any one of claims 1 to 3, wherein the rare earth element is europium (Eu), terbium (Tb), erbium (Er), ytterbium (Yb), neodymium (Nd) or samarium (Sm).
